# EUROPEAN PATENT APPLICATION

(11) **EP 4 620 478 A1**
(43) Date of publication of application: **24.09.2025**
(21) Application number: 25165292.1
(22) Date of filing: 21.03.2025
(51) Int. Cl.: A61K 35/19, A61K 9/00, A61K 9/19, A61M 1/02, A61M 1/36, A61P 11/00, A61P 29/00

(54) **PHARMACEUTICAL COMPOSITION COMPRISING PLATELET-DERIVED EXTRACELLULAR VESICLES OF DIFFERENT SIZES ACTIVATED BY FREEZE-THAW CYCLES, PREPARATION PROCESS THEREOF, AND USE IN PROMOTING WOUND HEALING OF RESPIRATORY TRACT-RELATED TISSUES AND ALLEVIATING INFLAMMA**

(30) Priority: 22.03.2024 US 202463568461 P; 17.03.2025 TW 114109931
(71) Applicant: Spirit Scientific Co. LTD., New Taipei City 221416 (TW); Lin, Dao Lung, Steven, New Taipei City 221416 (TW)
(72) Inventor: Lin, Dao-Lung Steven, 221416 New Taipei City (TW); Chen, Chin-Ho, 221416 New Taipei City (TW)
(74) Representative: Scholz, Volker

(57) **Abstract**

The present disclosure provides a pharmaceutical composition including a freeze and thaw activated platelet-derived extracellular vesicle with different sizes, the preparation process thereof and a use of the pharmaceutical composition for promoting wound healing of respiratory tract-related tissues and alleviating inflammation. The pharmaceutical composition of the present disclosure achieves the effect of promoting wound healing of respiratory tract-related tissues and alleviating inflammation through various efficacy experiments.

## Description

### BACKGROUND OF THE INVENTION

### 1. Field of the Invention

The present invention relates to a pharmaceutical composition comprising a freeze and thaw activated platelet-derived extracellular vesicle with different sizes, the preparation process thereof and a use of the pharmaceutical composition for promoting wound healing of respiratory tract-related tissues and alleviating inflammation.

### 2. The Prior Art

The administration of drugs via the respiratory system, known as "inhalation therapy," is primarily used for the treatment of respiratory tract and pulmonary diseases. The known characteristics of conventional inhalable formulations include non-invasive therapy, rapid drug effect, fewer side effects, and a lower required dosage.

The current types of inhalable formulations are classified as follows: 1. Metered-dose inhaler (MDI): The most common type of inhaler, which releases the medication in a pressurized spray upon pressing the delivery button. 2. Dry powder inhaler (DPI): Requires a "fast and deep" inhalation to effectively draw the powdered medication into the airways, minimizing residual drug retention. 3. Nebulizer: Converts liquid inhalation solutions into an aerosolized mist, allowing effective drug inhalation for users with poor hand-mouth coordination or insufficient respiratory strength.

However, these formulations mainly consist of chemically synthesized drugs, biologics, or cell-based therapies, which require special storage conditions and are inconvenient to use, often leading to side effects. Moreover, chemically synthesized drugs, biologics, and cell-based therapies are predominantly administered via injection, making them invasive treatments. Biologics and cell-based therapies are also expensive. Some drugs may lead to tolerance issues, necessitating higher dosages to maintain therapeutic efficacy. Additionally, the development of chemically synthesized drugs, biologics, and cell-based therapies is time-consuming and costly.

In order to solve the above-mentioned problems, those skilled in the art urgently need to develop a natural, non-synthetic, easy-to-use and easy-to-preserve inhalable formulation and its manufacturing method for the benefit of a large group of people in need thereof.

### SUMMARY OF THE INVENTION

A primary objective of the present invention is to provide a pharmaceutical composition, comprising a freeze and thaw activated platelet-derived extracellular vesicle, wherein the freeze and thaw activated platelet-derived extracellular vesicle is processed by size exclusion chromatography (SEC) to have different particle sizes.

Another objective of the present invention is to provide a method for preparing the aforementioned pharmaceutical composition, comprising the following steps: (a) centrifuging a human whole blood sample to separate a plasma layer and remove leukocytes and erythrocytes, thereby obtaining platelet-rich plasma (PRP); (b) centrifuging the platelet-rich plasma (PRP) to collect pellet and resuspending platelets in a solvent to obtain a platelet solution; (c) subjecting the platelet solution to repeated freeze-thaw cycles to obtain a freeze and thaw activated platelet solution; (d) centrifuging the freeze and thaw activated platelet solution and collecting supernatant to obtain a freeze and thaw activated platelet solution supernatant; and (e) filtering the freeze and thaw activated platelet solution supernatant to obtain the freeze-thaw activated platelet-derived extracellular vesicle, wherein the freeze and thaw activated platelet-derived extracellular vesicle is processed by size exclusion chromatography (SEC) to have different particle sizes.

According to an embodiment of the present invention, the freeze and thaw activated platelet-derived extracellular vesicle has a particle size ranging from 70-1,000 nm.

According to an embodiment of the present invention, the freeze and thaw activated platelet-derived extracellular vesicle has a particle size ranging from 35-400 nm.

According to an embodiment of the present invention, the freeze and thaw activated platelet-derived extracellular vesicle has a particle size ranging from 20-100 nm.

According to an embodiment of the present invention, the freeze and thaw activated platelet-derived extracellular vesicle is further subjected to a lyophilization procedure, thereby transforming the pharmaceutical composition into a form of an inhaler.

According to an embodiment of the present invention, the freeze and thaw activated platelet-derived extracellular vesicle is further subjected to a nebulization procedure, thereby transforming the pharmaceutical composition into a form of an inhaler.

According to an embodiment of the present invention, the inhaler is a metered-dose inhaler, a dry powder inhaler (DPI), or a nebulizer.

Another objective of the present invention is to provide a method for promoting wound healing of respiratory tract-related tissues and alleviating inflammation, comprising administering to a subject in need thereof the aforementioned pharmaceutical composition.

According to an embodiment of the present invention, the pharmaceutical composition can alleviate inflammation and accelerate wound healing of respiratory tract-related tissues by promoting cell migration and cell proliferation.

In summary, the present invention, as demonstrated by the results of the following examples, achieves the following technical effects: improving storage conditions; reducing side effects associated with chemically synthesized drugs, biologics, or cell-based therapies; addressing issues related to invasive treatments; overcoming usage limitations and regulatory restrictions of chemically synthesized drugs, biologics, or cell-based therapies; reducing the high costs of biologics or cell-based therapies; and improving the biological tolerance of natural biologics produced by cells and blood derivatives.

The means and improvements provided by the present invention are as follows. While chemically synthesized drugs, biologics, or cell-based therapies typically require strict storage conditions, the present invention can be stored under regular environmental conditions without special requirements. Chemically synthesized drugs and biologics are often artificially synthesized, raising concerns about potential side effects. In contrast, the present invention is a biologics product derived from cells and blood components, with no known side effects. Furthermore, while most chemically synthesized drugs, biologics, or cell-based therapies require invasive administration, the present invention is designed for inhalation, offering a non-invasive therapeutic approach. Conventional chemically synthesized drugs, biologics, and cell-based therapies can be difficult to use and any pose a risk of operational failure. In contrast, the present invention is easy to use and minimizes the risk of administration errors. Additionally, this biologic product can be efficiently mass-produced, thereby reducing costs. The present invention is a biologic product derived from cells and blood components, and has no problem of biological tolerance. Moreover, the present invention can be used in combination with other natural biologics produced from cells and blood derivatives to enhance therapeutic efficacy.

The embodiments of the present invention will be further described below. The following examples are used to illustrate the present invention and are not intended to limit the scope of the present invention. Anyone skilled in the art can make some changes and modifications without departing from the spirit and scope of the present invention. Therefore, the scope of the present invention shall be defined by the appended claims.

### BRIEF DESCRIPTION OF THE DRAWINGS

The following drawings form part of the present specification and are included here to further demonstrate some aspects of the present invention, which can be better understood by reference to one or more of these drawings, in combination with the detailed description of the embodiments presented herein.
FIG. 1 shows the concentration comparison of the platelet-derived extracellular vesicle with different particle sizes before and after nebulization.
FIG. 2A shows the ability of non-nebulized platelet-derived extracellular vesicles to promote cell migration.
FIG. 2B shows the ability of nebulized platelet-derived extracellular vesicles to promote cell migration.
FIG. 2C shows the ability of non-nebulized platelet-derived extracellular vesicles to promote cell proliferation.
FIG. 2D shows the ability of nebulized platelet-derived extracellular vesicles to promote cell proliferation.
FIG. 3 shows the comparison of the inflammatory index (IL-6) of platelet-derived extracellular vesicles with various particle sizes before and after nebulization.
FIG. 4 shows the comparison of the ability of platelet-derived extracellular vesicles with various particle sizes to promote cell migration with or without freeze-drying.
FIG. 5 shows the comparison of the ability of platelet-derived extracellular vesicles with various particle sizes to promote cell proliferation with or without freeze-drying.
FIG. 6 shows the comparison of the inflammatory index (IL-6) of platelet-derived extracellular vesicles with various particle sizes with or without freeze-drying.

### DETAILED DESCRIPTION OF THE PREFERRED EMBODIMENT

In the following detailed description of the embodiments of the present invention, reference is made to the accompanying drawings, which are shown to illustrate the specific embodiments in which the present disclosure may be practiced. These embodiments are provided to enable those skilled in the art to practice the present disclosure. It is understood that other embodiments may be used and that changes can be made to the embodiments without departing from the scope of the present invention. The following description is therefore not to be considered as limiting the scope of the present invention.

### Definition

As used herein, the data provided represent experimental values that can vary within a range of ±10%, preferably within ±5%.

Unless otherwise stated in the context, "a", "the" and similar terms used in the specification (especially in the following claims) should be understood as including singular and plural forms.

According to the present invention, extracellular vesicles (EVs) are small membrane-bound particles secreted by cells, ranging in size from 30 to 1,000 nm. Their primary function is to mediate intercellular communication. Nearly all cells release these lipid bilayer vesicles, which are non-replicable. EVs can carry a variety of biomolecules, including proteins, nucleic acids, lipids, metabolites, and even organelles from the originating cells. Their sources include human organ cells, animal cells, plants, or microorganisms. In the human body, various cells can produce exosomes which are filled in various biofluids, such as blood, saliva, amniotic fluid, placenta, joint capsule fluid, tumor cells, semen, sweat glands, urine, lymph, cerebrospinal fluid, etc.

Aerosol particles of different sizes would be deposited in different places in the respiratory tract. A small nebulizer generates aerosolized drug particles within the range of 0.5-5 µm, which can be deposited in the trachea and bronchi. Larger particles tend to settle in the mouth, nasal cavity, and throat, whereas excessively small particles may reach the alveoli or be exhaled before deposition.

In addition, because moisture would make aerosol particles larger, there would be fewer aerosol particles deposited in the trachea and bronchi. Therefore, the present invention uses platelets as a source to prepare a platelet-derived extracellular vesicle with different particle sizes, and overcomes the problem that the membrane structure of the extracellular vesicle is unstable, which makes it easy to be destroyed. Nebulizing the platelet-derived extracellular vesicle successfully and effectively solves the needs of being natural, non-synthetic, easy to use and easy to store.

According to the present invention, the respiratory tract-related tissues include, but are not limited to, nasal mucosa, trachea, and bronchi.

The present invention is further illustrated by the following examples. These examples are provided for illustration only and are not intended to limit the scope of the present invention. The scope of the present invention is shown in the appended claims.

### Example 1

### Concentration comparison of platelet-derived extracellular vesicle with different particle sizes before and after nebulization

The purpose of this example is to prepare a platelet-derived extracellular vesicle with different particle sizes and perform nebulization to confirm that the membrane structure can be stable through the nebulization process.

The experimental steps of this example are as follows. 250 mL of human whole blood was placed into a blood bag containing anticoagulant; a centrifuge was performed to centrifuge the human whole blood (500-1,200g, 5-8 minutes; this is the first centrifugation) to separate the human whole blood into three layers. From top to bottom, they are the plasma layer, the buffy coat layer, where white blood cells are located, and the red blood cells layer. The stratification of blood cells after centrifugation is well known to those with ordinary skills in the art, and the centrifugation conditions for the first centrifugation can be expanded to 300-1,500g, 3-10 minutes. After centrifugation, platelets would be distributed in the plasma layer and adjacent to the buffy coat layer. A sterile operation method was used to absorb the plasma layer to completely remove white blood cells and red blood cells. This is platelet-rich plasma (PRP), also known as plasma layer solution.

A portion of platelet-rich plasma (PRP) was collected and subjected to a second centrifugation at 1,000~2,500g for 5 minutes to pellet the platelets. The resulting pellet is the platelet layer, while the supernatant contains plasma and plasma proteins. The supernatant was completely removed to eliminate plasma and proteins in plasma, and the platelets in the platelet layer was resuspended using isotonic sodium chloride solution injection (brand: SinTong) to achieve a platelet concentration of 1×10⁹/mL (total volume of 10-20 mL), thereby obtaining a pure platelet solution. The platelet solution was frozen at -80°C (24 hours), thawed at 37°C (15 minutes), and the freeze-thaw cycle was repeated three times to obtain a freeze and thaw activated platelet solution. A centrifuge was performed to centrifuge the freeze and thaw activated platelet solution (10,000-15,000g, 5-10 minutes; this is the third centrifugation). The supernatant was collected to obtain the freeze and thaw activated platelet supernatant. The freeze and thaw activated platelet supernatant was filtered using a 0.45 µm syringe filter (PES material, 25 mm, model C0000296, brand: Labfil^{®}) to remove platelets and most impurities, thereby obtaining a freeze and thaw activated platelet-derived extracellular vesicle (EV).

Take a portion of the freeze and thaw activated platelet-derived extracellular vesicle and use different size exclusion chromatography (SEC) column (brand: IZON; qEV10 column) for size selection. The freeze and thaw activated platelet-derived extracellular vesicle is separated into three different size ranges: large particle size (70-1,000 nm), medium particle size (35-400 nm), and small particle size (20-100 nm). The freeze and thaw activated platelet-derived extracellular vesicles that have not undergone size exclusion chromatography is the freeze and thaw activated platelet-derived extracellular vesicle regardless of particle size.

An aerosol generator (model: Pulmogine, brand: HCmed) was used, the platelet-derived extracellular vesicle with various particle sizes and the platelet-derived extracellular vesicle that has not been subjected to SEC were used to perform a nebulization procedure, thereby obtaining multiple aerosols respectively. An aerosol generator can be one that complies with the quality management system (QMS) for medical devices or has been approved for market release by health authorities such as the FDA. Examples include the Pulmogine medical nebulizer (brand: HCmed), the NEB 800 medical nebulizer (brand: Microlife), or the TD-7001 medical nebulizer (brand: Clever Check). In this example, the aerosol generator used has a pore size of 5 µm and consists of two main components: the upper half of the component is the reservoir, and the lower half of the component is the main unit. The freeze and thaw activated platelet-derived extracellular vesicle (EV) is poured into the reservoir, which is then sealed with a reservoir cap. The reservoir is connected to the main unit, and a centrifuge tube is connected to the reservoir to collect the nebulized recovery liquid. Upon pressing the activation button on the main unit, the freeze and thaw activated platelet-derived EV is completely nebulized into aerosols.

The collected nebulized recovery liquid is analyzed using a tunable resistive pulse sensing (TRPS) nanoparticle analyzer (model: Exoid, brand: IZON Science) to quantify nanoparticles. The degree of nebulization is calculated as (post-nebulization concentration ÷ pre-nebulization concentration) × 100%.

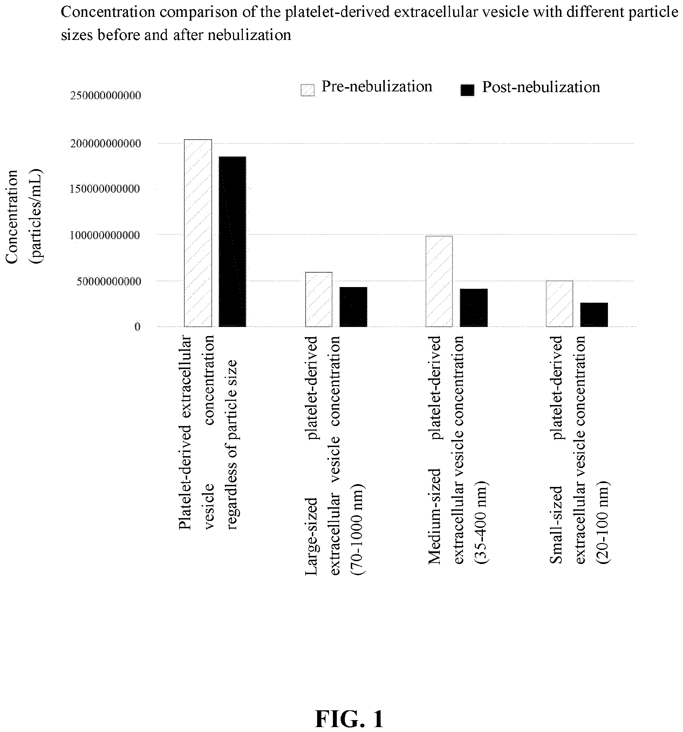

The experimental results are shown in Table 1 and FIG. 1.

**Table 1**

| Particles/mL | Pre-nebulization | Post-nebulization | Degree of nebulization |
|---|---|---|---|
| Platelet-derived extracellular vesicle concentration regardless of particle size | 204,000,000,000 | 185,000,000,000 | 91% |
| Large-sized platelet-derived extracellular vesicle concentration (70-1,000 nm) | 60,000,000,000 | 43,400,000,000 | 72% |
| Medium-sized platelet-derived extracellular vesicle concentration (35-400 nm) | 98,900,000,000 | 41,600,000,000 | 42% |
| Small-sized platelet-derived extracellular vesicle concentration (20-100 nm) | 50,700,000,000 | 26,200,000,000 | 52% |

As can be seen from Table 1 and FIG. 1, the degree of nebulization of the platelet-derived extracellular vesicle regardless of particle size is greater than that of large particle size, medium particle size, and small particle size.

### Example 2

### Healing test using platelet-derived extracellular vesicle with different particle sizes

The purpose of this example is to culture human nasal mucosal epithelial cells, platelet-derived extracellular vesicle with different particle sizes (final concentration 5%) was added, and a healing test (cell migration and cell proliferation) was conducted, observing whether there is any influence of nebulization.

### 2-1 Ability of non-nebulized platelet-derived extracellular vesicle to promote cell migration

The experimental steps are as follows. According to the preparation process in Example 1, four kinds of platelet-derived extracellular vesicle were prepared.

Human nasal mucosal epithelial cells (brand: Promocell, product number: C-12620) were cultured in Dulbecco's modified Eagle medium (DMEM) (Corning 10-013CV) containing 2% fetal bovine serum (FBS), and then replaced with fresh culture medium for later use.

A 24-well plate containing a cell culture insert is grouped into original wells, wells of platelet-derived extracellular vesicle group regardless of particle size, wells of large-sized platelet-derived extracellular vesicle group, wells of medium-sized platelet-derived extracellular vesicle group, and wells of small-sized platelet-derived extracellular vesicle group, 3 replicates per well. Human nasal mucosal epithelial cells were added to the wells in each group, allowing each well contains the same number of cells and culturing in an incubator at 37°C and 5% carbon dioxide (CO₂) for 24 hours.

The used medium and the cell culture intervals of each group were removed, and photos were taken and recorded with a microscope. Then, each group performs the following steps.

Original wells: DMEM culture medium (containing 2% FBS) was added and continually cultured for 6 hours in an incubator at 37°C and 5% carbon dioxide (CO₂).

Platelet-derived extracellular vesicle regardless of particle size group: The platelet-derived extracellular vesicle regardless of particle size and DMEM culture medium (containing 2% FBS) were mixed (volume ratio is 1:19) to prepare the platelet-derived extracellular vesicle culture medium regardless of particle size. Subsequently, the platelet-derived extracellular vesicle culture medium regardless of particle size was added to the wells of platelet-derived extracellular vesicle group regardless of particle size group and continually cultured for 6 hours in an incubator at 37°C and 5% carbon dioxide (CO₂).

Large-sized platelet-derived extracellular vesicle group: The large-sized platelet-derived extracellular vesicle and DMEM culture medium (containing 2% FBS) were mixed (volume ratio is 1:19) to prepare the large-sized platelet-derived extracellular vesicle culture medium. Subsequently, the large-sized platelet-derived extracellular vesicle culture medium was added to the wells of large-sized platelet-derived extracellular vesicle group, and continually cultured for 6 hours in an incubator at 37°C and 5% carbon dioxide (CO₂).

Medium-sized platelet-derived extracellular vesicle group: The medium-sized platelet-derived extracellular vesicle and DMEM culture medium (containing 2% FBS) were mixed (volume ratio is 1:19) to prepare the medium-sized platelet-derived extracellular vesicle culture medium. Subsequently, the medium-sized platelet-derived extracellular vesicle culture medium was added to the wells of medium-sized platelet-derived extracellular vesicle group, and continually cultured for 6 hours in an incubator at 37°C and 5% carbon dioxide (CO₂).

Small-sized platelet-derived extracellular vesicle group: The small-sized platelet-derived extracellular vesicle and DMEM culture medium (containing 2% FBS) were mixed (volume ratio is 1:19) to prepare the small-sized platelet-derived extracellular vesicle culture medium. Subsequently, the small-sized platelet-derived extracellular vesicle culture medium was added to the wells of small-sized platelet-derived extracellular vesicle group, and continually cultured for 6 hours in an incubator at 37°C and 5% carbon dioxide (CO₂).

Photos were taken and recorded with a microscope, and the cell migration coverage area of each group was calculated.

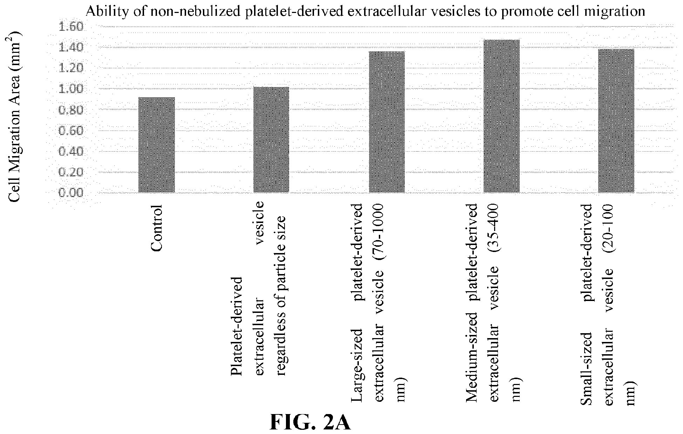

Experimental results are shown in Table 2 and FIG. 2A.

**Table 2**

| Control | Platelet-derived extracellular vesicle regardless of particle size | Large-sized platelet-derived extracellular vesicle (70-1000 nm) | Medium-sized platelet-derived extracellular vesicle (35-400 nm) | Small-sized platelet-derived extracellular vesicle (20-100 nm) |
|---|---|---|---|---|
| 0.92 | 1.02 | 1.36 | 1.48 | 1.38 |

As shown in Table 2 and FIG. 2A, among the non-nebulized groups, the medium-sized platelet-derived extracellular vesicles exhibited the greatest enhancement in cell migration.

### 2-2 Ability of nebulized platelet-derived extracellular vesicle to promote cell migration

The experimental steps are as follows. According to the preparation process in Example 1, four kinds of platelet-derived extracellular vesicle were prepared.

Each of the four types of platelet-derived extracellular vesicles was subjected to the nebulization process described in Example 1 using an aerosol generator (Pulmogine; brand HCmed), producing multiple aerosolized vesicles. In this experiment, a centrifuge tube is used to collect the nebulized aerosols, the aerosols are gathered into a recovery liquid in the centrifuge tube, and subsequent experiments are carried out.

Experiments were performed using the same human nasal mucosal epithelial cells as in Example 2-1.

A 24-well plate containing a cell culture insert is grouped into original wells, wells of platelet-derived extracellular vesicle nebulized group regardless of particle size, wells of large-sized platelet-derived extracellular vesicle nebulized group, wells of medium-sized platelet-derived extracellular vesicle nebulized group, and wells of small-sized platelet-derived extracellular vesicle nebulized group, 3 replicates per well. Human nasal mucosal epithelial cells were added to the wells in each group, allowing each well contains the same number of cells and culturing in an incubator at 37°C and 5% carbon dioxide (CO₂) for 24 hours.

The used medium and the cell culture insert of each group were removed, and photos were taken and recorded with a microscope. Then, each group performs the following steps.

Original wells: DMEM culture medium (containing 2% FBS) was added and continually cultured for 6 hours in an incubator at 37°C and 5% carbon dioxide (CO₂).

Platelet-derived extracellular vesicle nebulized regardless of particle size group: The platelet-derived extracellular vesicle nebulized recovery liquid regardless of particle size and DMEM culture medium (containing 2% FBS) were mixed (volume ratio is 1:19) to prepare the platelet-derived extracellular vesicle nebulized culture medium regardless of particle size. Subsequently, the platelet-derived extracellular vesicle nebulized culture medium regardless of particle size was added to the wells of platelet-derived extracellular vesicle nebulized group regardless of particle size group and continually cultured for 6 hours in an incubator at 37°C and 5% carbon dioxide (CO₂).

Large-sized platelet-derived extracellular vesicle nebulized group: The large-sized platelet-derived extracellular vesicle nebulized recovery liquid and DMEM culture medium (containing 2% FBS) were mixed (volume ratio is 1:19) to prepare the large-sized platelet-derived extracellular vesicle nebulized culture medium. Subsequently, the large-sized platelet-derived extracellular vesicle nebulized culture medium was added to the wells of large-sized platelet-derived extracellular vesicle nebulized group, and continually cultured for 6 hours in an incubator at 37°C and 5% carbon dioxide (CO₂).

Medium-sized platelet-derived extracellular vesicle nebulized group: The medium-sized platelet-derived extracellular vesicle nebulized recovery liquid and DMEM culture medium (containing 2% FBS) were mixed (volume ratio is 1:19) to prepare the medium-sized platelet-derived extracellular vesicle nebulized culture medium. Subsequently, the medium-sized platelet-derived extracellular vesicle nebulized culture medium was added to the wells of medium-sized platelet-derived extracellular vesicle nebulized group, and continually cultured for 6 hours in an incubator at 37°C and 5% carbon dioxide (CO₂).

Small-sized platelet-derived extracellular vesicle nebulized group: The small-sized platelet-derived extracellular vesicle nebulized recovery liquid and DMEM culture medium (containing 2% FBS) were mixed (volume ratio is 1:19) to prepare the small-sized platelet-derived extracellular vesicle nebulized culture medium. Subsequently, the small-sized platelet-derived extracellular vesicle nebulized culture medium was added to the wells of small-sized platelet-derived extracellular vesicle nebulized group, and continually cultured for 6 hours in an incubator at 37°C and 5% carbon dioxide (CO₂).

Photos were taken and recorded with a microscope, and the cell migration coverage area of each group was calculated.

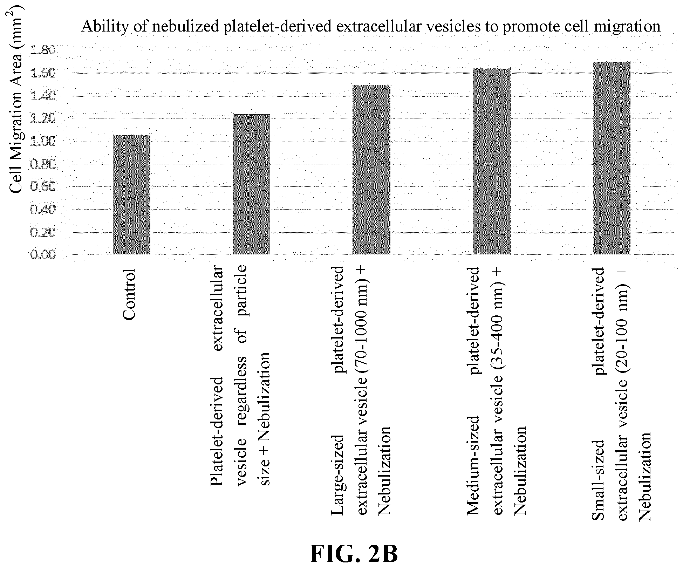

Experimental results are shown in Table 3 and FIG. 2B.

**Table 3**

| Control | Platelet-derived extracellular vesicle regardless of particle size + Nebulization | Large-sized platelet-derived extracellular vesicle (70-1000 nm) + Nebulization | Medium-sized platelet-derived extracellular vesicle (35-400 nm) + Nebulization | Small-sized platelet-derived extracellular vesicle (20-100 nm) + Nebulization |
|---|---|---|---|---|
| 1.05 | 1.24 | 1.50 | 1.64 | 1.70 |

As can be seen from Table 3 and FIG. 2B, the difference in cell migration area with the nebulization process is that the effect of small-sized platelet-derived extracellular vesicle is the best.

### 2-3 Ability of non-nebulized platelet-derived extracellular vesicle to promote cell proliferation

The experimental steps are as follows. According to the preparation process in Example 1, four kinds of platelet-derived extracellular vesicle were prepared.

Experiments were performed using the same human nasal mucosal epithelial cells and platelet-derived extracellular vesicle as in Example 2-1.

A 24-well plate is grouped into original wells, wells of large-sized platelet-derived extracellular vesicle group, wells of medium-sized platelet-derived extracellular vesicle group, and wells of small-sized platelet-derived extracellular vesicle group, 3 replicates per well. Human nasal mucosal epithelial cells were added to the wells in each group, allowing each well contains the same number of cells and culturing in an incubator at 37°C and 5% carbon dioxide (CO₂) for 24 hours. Then, each group performs the following steps.

The used medium was removed, and the following steps were performed.

Original wells: DMEM culture medium (containing 2% FBS) was added into the original wells, and continually cultured for 18 hours in an incubator at 37°C and 5% carbon dioxide (CO₂).

Large-sized platelet-derived extracellular vesicle group: The large-sized platelet-derived extracellular vesicle and DMEM culture medium (containing 2% FBS) were mixed (volume ratio is 1:19) to prepare the large-sized platelet-derived extracellular vesicle culture medium. Subsequently, the large-sized platelet-derived extracellular vesicle culture medium was added to the wells of large-sized platelet-derived extracellular vesicle group, and continually cultured for 18 hours in an incubator at 37°C and 5% carbon dioxide (CO₂).

Medium-sized platelet-derived extracellular vesicle group: The medium-sized platelet-derived extracellular vesicle and DMEM culture medium (containing 2% FBS) were mixed (volume ratio is 1:19) to prepare the medium-sized platelet-derived extracellular vesicle culture medium. Subsequently, the medium-sized platelet-derived extracellular vesicle culture medium was added to the wells of medium-sized platelet-derived extracellular vesicle group, and continually cultured for 18 hours in an incubator at 37°C and 5% carbon dioxide (CO₂).

Small-sized platelet-derived extracellular vesicle group: The small-sized platelet-derived extracellular vesicle and DMEM culture medium (containing 2% FBS) were mixed (volume ratio is 1:19) to prepare the small-sized platelet-derived extracellular vesicle culture medium. Subsequently, the small-sized platelet-derived extracellular vesicle culture medium was added to the wells of small-sized platelet-derived extracellular vesicle group, and continually cultured for 18 hours in an incubator at 37°C and 5% carbon dioxide (CO₂).

The used medium of each group was removed. 90 µL of DMEM culture medium and 10 µL of CCK-8 buffer (DOJINDO, product number CK04) were added to each well, followed by continually culturing for 3 hours in an incubator at 37°C and 5% carbon dioxide (CO₂).

A multifunctional microplate reader (model: Varioskan LUX^{™}, brand: Thermo Scientific^{™}) was used to measure absorbance at 450 nm. A standard curve correlating absorbance value with cell numbers was established based on the CCK-8 buffer product instructions, and the absorbance value were converted into the viable cell counts.

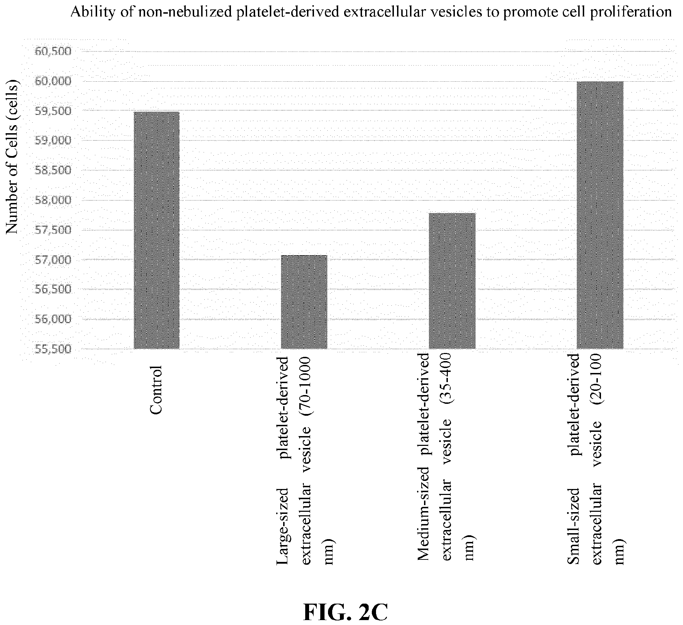

Experimental results are shown in Table 4 and FIG. 2C.

**Table 4**

| Control | Large-sized platelet-derived extracellular vesicle (70-1000 nm) | Medium-sized platelet-derived extracellular vesicle (35-400 nm) | Small-sized platelet-derived extracellular vesicle (20-100 nm) |
|---|---|---|---|
| 59,480 | 57,086 | 57,780 | 59,995 |

As can be seen from Table 4 and FIG. 2C, the cell proliferation status without the nebulization process shows that the cell proliferation effect of the small-sized platelet-derived extracellular vesicle is the best.

### 2-4 Ability of nebulized platelet-derived extracellular vesicle to promote cell proliferation

The experimental steps are as follows. According to the preparation process in Example 1, four kinds of platelet-derived extracellular vesicle were prepared and nebulized.

Each of the four types of platelet-derived extracellular vesicle was subjected to the nebulization process described in Example 1 using an aerosol generator (Pulmogine; brand HCmed), producing multiple aerosolized vesicles. In this experiment, a centrifuge tube is used to collect the nebulized aerosols, the aerosols are gathered into a recovery liquid in the centrifuge tube, and subsequent experiments are carried out.

Experiments were performed using the same human nasal mucosal epithelial cells and platelet-derived extracellular vesicle as in Example 2-1.

A 24-well plate is grouped into original wells, wells of large-sized platelet-derived extracellular vesicle nebulized group, wells of medium-sized platelet-derived extracellular vesicle nebulized group, and wells of small-sized platelet-derived extracellular vesicle nebulized group, 3 replicates per well. Human nasal mucosal epithelial cells were added to the wells in each group, allowing each well contains the same number of cells and culturing in an incubator at 37°C and 5% carbon dioxide (CO₂) for 24 hours. Then, each group performs the following steps.

The used medium was removed, and the following steps were performed.

Original wells: DMEM culture medium (containing 2% FBS) was added into the original wells, and continually cultured for 18 hours in an incubator at 37°C and 5% carbon dioxide (CO₂).

Large-sized platelet-derived extracellular vesicle nebulized group: The large-sized platelet-derived extracellular vesicle nebulized recovery liquid and DMEM culture medium (containing 2% FBS) were mixed (volume ratio is 1:19) to prepare the large-sized platelet-derived extracellular vesicle nebulized culture medium. Subsequently, the large-sized platelet-derived extracellular vesicle nebulized culture medium was added to the wells of large-sized platelet-derived extracellular vesicle nebulized group, and continually cultured for 18 hours in an incubator at 37°C and 5% carbon dioxide (CO₂).

Medium-sized platelet-derived extracellular vesicle nebulized group: The medium-sized platelet-derived extracellular vesicle nebulized recovery liquid and DMEM culture medium (containing 2% FBS) were mixed (volume ratio is 1:19) to prepare the medium-sized platelet-derived extracellular vesicle nebulized culture medium. Subsequently, the medium-sized platelet-derived extracellular vesicle nebulized culture medium was added to the wells of medium-sized platelet-derived extracellular vesicle nebulized group, and continually cultured for 18 hours in an incubator at 37°C and 5% carbon dioxide (CO₂).

Small-sized platelet-derived extracellular vesicle nebulized group: The small-sized platelet-derived extracellular vesicle nebulized recovery liquid and DMEM culture medium (containing 2% FBS) were mixed (volume ratio is 1:19) to prepare the small-sized platelet-derived extracellular vesicle nebulized culture medium. Subsequently, the small-sized platelet-derived extracellular vesicle nebulized culture medium was added to the wells of small-sized platelet-derived extracellular vesicle nebulized group, and continually cultured for 18 hours in an incubator at 37°C and 5% carbon dioxide (CO₂).

The used medium of each group was removed. 90 µL of DMEM culture medium and 10 µL of CCK-8 buffer (DOJINDO, product number CK04) were added to each well, followed by continually culturing for 3 hours in an incubator at 37°C and 5% carbon dioxide (CO₂).

A multifunctional microplate reader (model: Varioskan LUX^{™}, brand: Thermo Scientific^{™}) was used to measure absorbance at 450 nm. A regression curve was established between absorbance value and cell number according to the CCK-8 buffer product instructions, and the absorbance value was then converted into the number of viable cells.

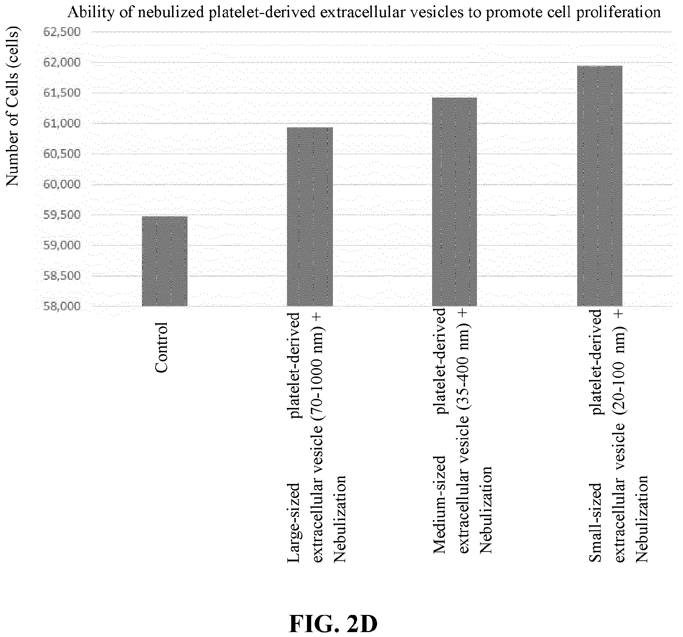

Experimental results are shown in Table 5 and FIG. 2D.

**Table 5**

| Control | Large-sized platelet-derived extracellular vesicle (70-1000 nm) + Nebulization | Medium-sized platelet-derived extracellular vesicle (35-400 nm) + Nebulization | Small-sized platelet-derived extracellular vesicle (20-100 nm) + Nebulization |
|---|---|---|---|
| 59,480 | 60,934 | 61,426 | 61,946 |

As shown in Table 5 and FIG. 2D, among the nebulized groups, the small-sized platelet-derived extracellular vesicles (20-100 nm) exhibited the greatest enhancement in cell proliferation.

### Example 3

### Comparison of ability of nebulized or non-nebulized platelet-derived extracellular vesicles to alleviate inflammation

The experimental steps are as follows. According to the preparation process in Example 1, four kinds of platelet-derived extracellular vesicle solution were prepared, and divided into two parts, one part of which was nebulized.

Each of the four types of platelet-derived extracellular vesicles was subjected to the nebulization process described in Example 1 using an aerosol generator (Pulmogine; brand HCmed), producing multiple aerosolized vesicles. In this experiment, a centrifuge tube is used to collect the nebulized aerosols, the aerosols are gathered into a recovery liquid in the centrifuge tube, and subsequent experiments are carried out.

Experiments were performed using the same human nasal mucosal epithelial cells as in Example 2-1.

A 96-well plate is grouped into original wells, wells of LPS control, wells of platelet-derived extracellular vesicle group regardless of particle size, wells of large-sized platelet-derived extracellular vesicle group, wells of medium-sized platelet-derived extracellular vesicle group, and wells of small-sized platelet-derived extracellular vesicle group, wells of platelet-derived extracellular vesicle nebulized group regardless of particle size, wells of large-sized platelet-derived extracellular vesicle nebulized group, wells of medium-sized platelet-derived extracellular vesicle nebulized group, and wells of small-sized platelet-derived extracellular vesicle nebulized group, 3 replicates per well. Human nasal mucosal epithelial cells were added to the wells in each group, allowing each well contains the same number of cells and culturing in an incubator at 37°C and 5% carbon dioxide (CO₂) for 24 hours.

The used medium was removed. The cells in the wells of LPS control and experimental wells were suspended in DMEM culture medium containing 5 µg/mL LPS (containing 2% FBS), and the cells in the original wells were suspended in DMEM culture medium without LPS (containing 2% FBS), followed by culturing for 1 hour in an incubator at 37°C and 5% carbon dioxide (CO₂).

The used medium was removed and the following steps were performed.

Original wells and LPS control wells. DMEM culture medium (containing 2% FBS) was added and continually cultured for 24 hours in an incubator at 37°C and 5% carbon dioxide (CO₂).

Platelet-derived extracellular vesicle regardless of particle size group: The platelet-derived extracellular vesicle regardless of particle size and DMEM culture medium (containing 2% FBS) were mixed (volume ratio is 1:19) to prepare the platelet-derived extracellular vesicle culture medium regardless of particle size. Subsequently, the platelet-derived extracellular vesicle culture medium regardless of particle size was added to the wells of platelet-derived extracellular vesicle group regardless of particle size group and continually cultured for 24 hours in an incubator at 37°C and 5% carbon dioxide (CO₂).

Large-sized platelet-derived extracellular vesicle group: The large-sized platelet-derived extracellular vesicle and DMEM culture medium (containing 2% FBS) were mixed (volume ratio is 1:19) to prepare the large-sized platelet-derived extracellular vesicle culture medium. Subsequently, the large-sized platelet-derived extracellular vesicle culture medium was added to the wells of large-sized platelet-derived extracellular vesicle group, and continually cultured for 24 hours in an incubator at 37°C and 5% carbon dioxide (CO₂).

Medium-sized platelet-derived extracellular vesicle group: The medium-sized platelet-derived extracellular vesicle and DMEM culture medium (containing 2% FBS) were mixed (volume ratio is 1:19) to prepare the medium-sized platelet-derived extracellular vesicle culture medium. Subsequently, the medium-sized platelet-derived extracellular vesicle culture medium was added to the wells of medium-sized platelet-derived extracellular vesicle group, and continually cultured for 24 hours in an incubator at 37°C and 5% carbon dioxide (CO₂).

Small-sized platelet-derived extracellular vesicle group: The small-sized platelet-derived extracellular vesicle and DMEM culture medium (containing 2% FBS) were mixed (volume ratio is 1:19) to prepare the small-sized platelet-derived extracellular vesicle culture medium. Subsequently, the small-sized platelet-derived extracellular vesicle culture medium was added to the wells of small-sized platelet-derived extracellular vesicle group, and continually cultured for 24 hours in an incubator at 37°C and 5% carbon dioxide (CO₂).

Platelet-derived extracellular vesicle nebulized regardless of particle size group: The platelet-derived extracellular vesicle nebulized recovery liquid regardless of particle size and DMEM culture medium (containing 2% FBS) were mixed (volume ratio is 1:19) to prepare the platelet-derived extracellular vesicle nebulized culture medium regardless of particle size. Subsequently, the platelet-derived extracellular vesicle nebulized culture medium regardless of particle size was added to the wells of platelet-derived extracellular vesicle nebulized group regardless of particle size group and continually cultured for 24 hours in an incubator at 37°C and 5% carbon dioxide (CO₂).

Large-sized platelet-derived extracellular vesicle nebulized group: The large-sized platelet-derived extracellular vesicle nebulized recovery liquid and DMEM culture medium (containing 2% FBS) were mixed (volume ratio is 1:19) to prepare the large-sized platelet-derived extracellular vesicle nebulized culture medium. Subsequently, the large-sized platelet-derived extracellular vesicle nebulized culture medium was added to the wells of large-sized platelet-derived extracellular vesicle nebulized group, and continually cultured for 24 hours in an incubator at 37°C and 5% carbon dioxide (CO₂).

Medium-sized platelet-derived extracellular vesicle nebulized group: The medium-sized platelet-derived extracellular vesicle nebulized recovery liquid and DMEM culture medium (containing 2% FBS) were mixed (volume ratio is 1:19) to prepare the medium-sized platelet-derived extracellular vesicle nebulized culture medium. Subsequently, the medium-sized platelet-derived extracellular vesicle nebulized culture medium was added to the wells of medium-sized platelet-derived extracellular vesicle nebulized group, and continually cultured for 24 hours in an incubator at 37°C and 5% carbon dioxide (CO₂).

Small-sized platelet-derived extracellular vesicle nebulized group: The small-sized platelet-derived extracellular vesicle nebulized recovery liquid and DMEM culture medium (containing 2% FBS) were mixed (volume ratio is 1:19) to prepare the small-sized platelet-derived extracellular vesicle nebulized culture medium. Subsequently, the small-sized platelet-derived extracellular vesicle nebulized culture medium was added to the wells of small-sized platelet-derived extracellular vesicle nebulized group, and continually cultured for 24 hours in an incubator at 37°C and 5% carbon dioxide (CO₂).

The cell culture medium in each well was transferred into a microcentrifuge tube. The interleukin (IL-6, an inflammatory factor) ELISA kit (human IL-6 ELISA kit) (Product No. ab178013, brand: Abcam) was used to detect the concentration of human IL-6 in the cell culture medium. A multifunctional micro-plate spectrometer (model: Varioskan LUX^{™}, brand: Thermo Scientific^{™}) was used for analysis, and the absorbance value was set to 450 nm. A regression curve was established between absorbance value and concentration of inflammatory factor IL-6 according to the interleukin (IL-6) ELISA kit product instructions, and the absorbance value was then converted into the concentration of inflammatory factor IL-6.

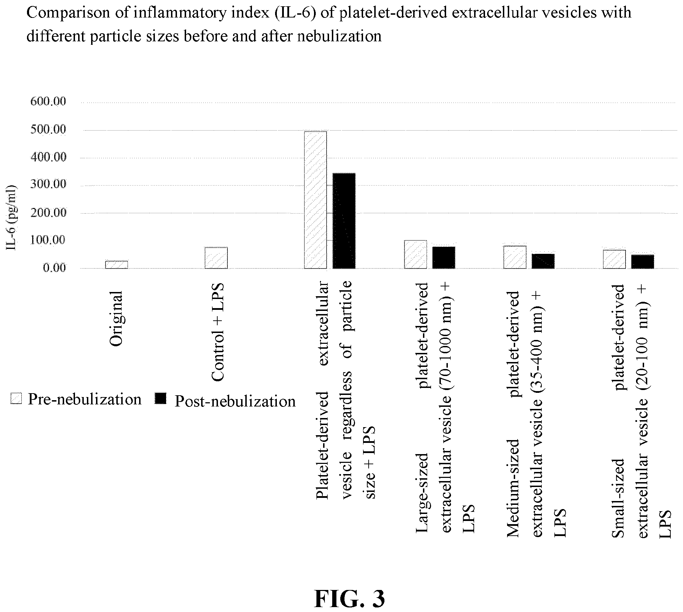

Experimental results are shown in Table 6 and FIG. 3.

**Table 6**

| **[0114]** As can be seen from Table 6 and FIG. 3, the nebulized small-sized | | | | | | | | | |
|---|---|---|---|---|---|---|---|---|---|
| Original | LPS control | Extracellular vesicle concentration regardless of particle size + LPS | Large-sized extracellular vesicle concentration (70-1000nm) + LPS | Medium-sized extracellular vesicle concentration (35-400nm) + LPS | Small-sized extracellular vesicle concentration (20-100nm) + LPS | Extracellular vesicle concentration regardless of particle size + LPS + Nebulization | Large-sized extracellular vesicle concentration (70-1000nm) + LPS + Nebulization | Medium-sized extracellular vesicle concentration (35-400nm) + LPS + Nebulization | Small-sized extracellular vesicle concentration (20-100nm) + LPS + Nebulization |
| 25.83 | 75.33 | 494.53 | 102.13 | 79.67 | 64.53 | 345.33 | 74.47 | 54.40 | 46.20 |

platelet-derived extracellular vesicle (20-100 nm) has the best ability to alleviate inflammation.

### Example 4

### Healing test using platelet-derived extracellular vesicle with or without freeze-drying

### 4-1 Comparison of ability of platelet-derived extracellular vesicles to promote cell migration with or without freeze-drying

The experimental steps are as follows. According to the preparation process in Example 1, four kinds of platelet-derived extracellular vesicle were prepared, and divided into two parts.

A portion of each of the four kinds of platelet-derived extracellular vesicle mentioned above was taken and freeze-dried in a volume of 1 mL to prepare four kinds of freeze and thaw activated platelet derived extracellular vesicle dry powder.

One portion of each of the four kinds of freeze and thaw activated platelet derived extracellular vesicle dry powder was taken and re-dissolved in 1 mL of normal saline to prepare four groups of freeze and thaw activated platelet derived extracellular vesicle dry powder solution.

Each group of platelet-derived extracellular vesicle was subjected to the nebulization procedure as described in Example 1 above using an aerosol generator (Pulmogine; brand HCmed), thereby obtaining multiple aerosols respectively. In this experiment, a centrifuge tube is used to collect the nebulized aerosols, the aerosols are gathered into a recovery liquid in the centrifuge tube, and subsequent experiments are carried out.

Experiments were performed using the same human nasal mucosal epithelial cells as in Example 2-1.

A 24-well plate containing a cell culture insert is grouped into wells of control group (original), wells of platelet-derived extracellular vesicle nebulized group regardless of particle size, wells of large-sized platelet-derived extracellular vesicle nebulized group, wells of medium-sized platelet-derived extracellular vesicle nebulized group, and wells of small-sized platelet-derived extracellular vesicle nebulized group; wells of platelet-derived extracellular vesicle freeze-dried nebulized group regardless of particle size, wells of large-sized platelet-derived extracellular vesicle freeze-dried nebulized group, wells of medium-sized platelet-derived extracellular vesicle freeze-dried nebulized group, and wells of small-sized platelet-derived extracellular vesicle freeze-dried nebulized group, 3 replicates per well. Human nasal mucosal epithelial cells were added to the wells in each group, allowing each well contains the same number of cells and culturing in an incubator at 37°C and 5% carbon dioxide (CO₂) for 24 hours.

The used medium and the cell culture insert of each group were removed, and photos were taken and recorded with a microscope. Then, each group performs the following steps.

Wells of control group (original): DMEM culture medium (containing 2% FBS) was added and continually cultured for 6 hours in an incubator at 37°C and 5% carbon dioxide (CO₂).

Platelet-derived extracellular vesicle nebulized regardless of particle size group: The platelet-derived extracellular vesicle nebulized recovery liquid regardless of particle size and DMEM culture medium (containing 2% FBS) were mixed (volume ratio is 1:19) to prepare the platelet-derived extracellular vesicle nebulized culture medium regardless of particle size. Subsequently, the platelet-derived extracellular vesicle nebulized culture medium regardless of particle size was added to the wells of platelet-derived extracellular vesicle nebulized group regardless of particle size group and continually cultured for 6 hours in an incubator at 37°C and 5% carbon dioxide (CO₂).

Large-sized platelet-derived extracellular vesicle nebulized group: The large-sized platelet-derived extracellular vesicle nebulized recovery liquid and DMEM culture medium (containing 2% FBS) were mixed (volume ratio is 1:19) to prepare the large-sized platelet-derived extracellular vesicle nebulized culture medium. Subsequently, the large-sized platelet-derived extracellular vesicle nebulized culture medium was added to the wells of large-sized platelet-derived extracellular vesicle nebulized group, and continually cultured for 6 hours in an incubator at 37°C and 5% carbon dioxide (CO₂).

Medium-sized platelet-derived extracellular vesicle nebulized group: The medium-sized platelet-derived extracellular vesicle nebulized recovery liquid and DMEM culture medium (containing 2% FBS) were mixed (volume ratio is 1:19) to prepare the medium-sized platelet-derived extracellular vesicle nebulized culture medium. Subsequently, the medium-sized platelet-derived extracellular vesicle nebulized culture medium was added to the wells of medium-sized platelet-derived extracellular vesicle nebulized group, and continually cultured for 6 hours in an incubator at 37°C and 5% carbon dioxide (CO₂).

Small-sized platelet-derived extracellular vesicle nebulized group: The small-sized platelet-derived extracellular vesicle nebulized recovery liquid and DMEM culture medium (containing 2% FBS) were mixed (volume ratio is 1:19) to prepare the small-sized platelet-derived extracellular vesicle nebulized culture medium. Subsequently, the small-sized platelet-derived extracellular vesicle nebulized culture medium was added to the wells of small-sized platelet-derived extracellular vesicle nebulized group, and continually cultured for 6 hours in an incubator at 37°C and 5% carbon dioxide (CO₂).

Platelet-derived extracellular vesicle freeze-dried nebulized regardless of particle size group: The nebulized recovery liquid of the platelet-derived extracellular vesicle regardless of particle size dry powder solution and DMEM culture medium (containing 2% FBS) were mixed (volume ratio is 1:19) to prepare the platelet-derived extracellular vesicle freeze-dried nebulized culture medium regardless of particle size. Subsequently, the platelet-derived extracellular vesicle freeze-dried nebulized culture medium regardless of particle size was added to the wells of platelet-derived extracellular vesicle freeze-dried nebulized group regardless of particle size group and continually cultured for 6 hours in an incubator at 37°C and 5% carbon dioxide (CO₂).

Large-sized platelet-derived extracellular vesicle freeze-dried nebulized group: The nebulized recovery liquid of the large-sized platelet-derived extracellular vesicle dry powder solution and DMEM culture medium (containing 2% FBS) were mixed (volume ratio is 1:19) to prepare the large-sized platelet-derived extracellular vesicle freeze-dried nebulized culture medium. Subsequently, the large-sized platelet-derived extracellular vesicle freeze-dried nebulized culture medium was added to the wells of large-sized platelet-derived extracellular vesicle freeze-dried nebulized group, and continually cultured for 6 hours in an incubator at 37°C and 5% carbon dioxide (CO₂).

Medium-sized platelet-derived extracellular vesicle freeze-dried nebulized group: The nebulized recovery liquid of the medium-sized platelet-derived extracellular vesicle dry powder solution and DMEM culture medium (containing 2% FBS) were mixed (volume ratio is 1:19) to prepare the medium-sized platelet-derived extracellular vesicle freeze-dried nebulized culture medium. Subsequently, the medium-sized platelet-derived extracellular vesicle freeze-dried nebulized culture medium was added to the wells of medium-sized platelet-derived extracellular vesicle freeze-dried nebulized group, and continually cultured for 6 hours in an incubator at 37°C and 5% carbon dioxide (CO₂).

Small-sized platelet-derived extracellular vesicle freeze-dried nebulized group: The nebulized recovery liquid of the small-sized platelet-derived extracellular vesicle dry powder solution and DMEM culture medium (containing 2% FBS) were mixed (volume ratio is 1:19) to prepare the small-sized platelet-derived extracellular vesicle freeze-dried nebulized culture medium. Subsequently, the small-sized platelet-derived extracellular vesicle freeze-dried nebulized culture medium was added to the wells of small-sized platelet-derived extracellular vesicle freeze-dried nebulized group, and continually cultured for 6 hours in an incubator at 37°C and 5% carbon dioxide (CO₂).

Photos were taken and recorded with a microscope, and the cell migration coverage area of each group was calculated.

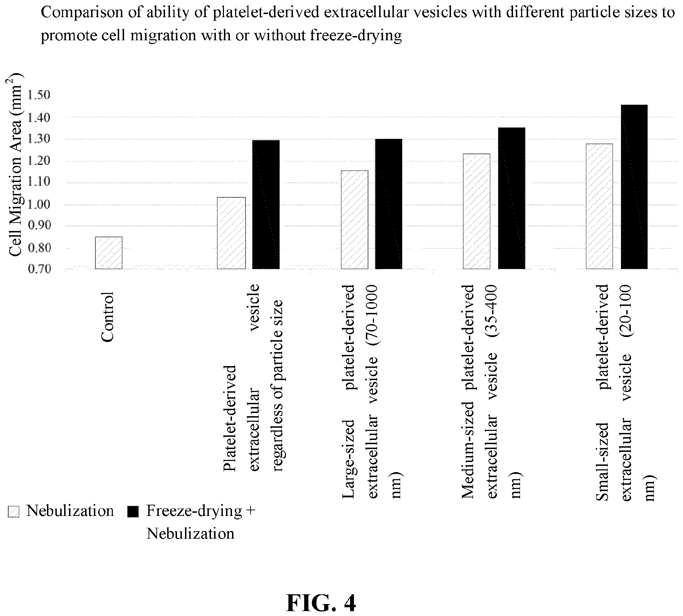

Experimental results are shown in Table 7 and FIG. 4.

**Table 7**

| | Control | Platelet-derived extracellular vesicle regardless of particle size | Large-sized platelet-derived extracellular vesicle (70-1000 nm) | Medium-sized platelet-derived extracellular vesicle (35-400 nm) | Small-sized platelet-derived extracellular vesicle (20-100 nm) |
|---|---|---|---|---|---|
| Nebulization | 0.85 | 1.03 | 1.15 | 1.23 | 1.28 |
| Freeze drying+ Nebulization | | 1.29 | 1.30 | 1.35 | 1.46 |

As can be seen from Table 7 and FIG. 4, the freeze-dried and nebulized platelet-derived extracellular vesicle has the best ability to promote cell migration.

### 4-2 Comparison of ability of platelet-derived extracellular vesicles to promote cell proliferation with or without freeze-drying

The experimental steps are as follows. According to the preparation process in Example 1, four kinds of platelet-derived extracellular vesicle were prepared, and divided into two parts.

A portion of each of the four kinds of platelet-derived extracellular vesicle mentioned above was taken and freeze-dried in a volume of 1 mL to prepare four kinds of freeze and thaw activated platelet derived extracellular vesicle dry powder.

One portion of each of the four kinds of freeze and thaw activated platelet derived extracellular vesicle dry powder was taken and re-dissolved in 1 mL of normal saline to prepare four groups of freeze and thaw activated platelet derived extracellular vesicle dry powder solution.

Each group of platelet-derived extracellular vesicle was subjected to the nebulization procedure as described in Example 1 above using an aerosol generator (Pulmogine; brand HCmed), thereby obtaining multiple aerosols respectively. In this experiment, a centrifuge tube is used to collect the nebulized aerosols, the aerosols are gathered into a recovery liquid in the centrifuge tube, and subsequent experiments are carried out.

Experiments were performed using the same human nasal mucosal epithelial cells as in Example 2-1.

A 24-well plate is grouped into wells of control group (original), wells of platelet-derived extracellular vesicle nebulized group regardless of particle size, wells of large-sized platelet-derived extracellular vesicle nebulized group, wells of medium-sized platelet-derived extracellular vesicle nebulized group, and wells of small-sized platelet-derived extracellular vesicle nebulized group; wells of platelet-derived extracellular vesicle freeze-dried nebulized group regardless of particle size, wells of large-sized platelet-derived extracellular vesicle freeze-dried nebulized group, wells of medium-sized platelet-derived extracellular vesicle freeze-dried nebulized group, and wells of small-sized platelet-derived extracellular vesicle freeze-dried nebulized group, 3 replicates per well. Human nasal mucosal epithelial cells were added to the wells in each group, allowing each well contains the same number of cells and culturing in an incubator at 37°C and 5% carbon dioxide (CO₂) for 24 hours. Then, each group performs the following steps.

The used medium was removed, and the following steps were performed.

Original wells and LPS control wells: DMEM culture medium (containing 2% FBS) was added and continually cultured for 18 hours in an incubator at 37°C and 5% carbon dioxide (CO₂).

Platelet-derived extracellular vesicle nebulized regardless of particle size group: The platelet-derived extracellular vesicle nebulized recovery liquid regardless of particle size and DMEM culture medium (containing 2% FBS) were mixed (volume ratio is 1:19) to prepare the platelet-derived extracellular vesicle nebulized culture medium regardless of particle size. Subsequently, the platelet-derived extracellular vesicle nebulized culture medium regardless of particle size was added to the wells of platelet-derived extracellular vesicle nebulized group regardless of particle size group and continually cultured for 18 hours in an incubator at 37°C and 5% carbon dioxide (CO₂).

Large-sized platelet-derived extracellular vesicle nebulized group: The large-sized platelet-derived extracellular vesicle nebulized recovery liquid and DMEM culture medium (containing 2% FBS) were mixed (volume ratio is 1:19) to prepare the large-sized platelet-derived extracellular vesicle nebulized culture medium. Subsequently, the large-sized platelet-derived extracellular vesicle nebulized culture medium was added to the wells of large-sized platelet-derived extracellular vesicle nebulized group, and continually cultured for 18 hours in an incubator at 37°C and 5% carbon dioxide (CO₂).

Medium-sized platelet-derived extracellular vesicle nebulized group: The medium-sized platelet-derived extracellular vesicle nebulized recovery liquid and DMEM culture medium (containing 2% FBS) were mixed (volume ratio is 1:19) to prepare the medium-sized platelet-derived extracellular vesicle nebulized culture medium. Subsequently, the medium-sized platelet-derived extracellular vesicle nebulized culture medium was added to the wells of medium-sized platelet-derived extracellular vesicle nebulized group, and continually cultured for 18 hours in an incubator at 37°C and 5% carbon dioxide (CO₂).

Small-sized platelet-derived extracellular vesicle nebulized group: The small-sized platelet-derived extracellular vesicle nebulized recovery liquid and DMEM culture medium (containing 2% FBS) were mixed (volume ratio is 1:19) to prepare the small-sized platelet-derived extracellular vesicle nebulized culture medium. Subsequently, the small-sized platelet-derived extracellular vesicle nebulized culture medium was added to the wells of small-sized platelet-derived extracellular vesicle nebulized group, and continually cultured for 18 hours in an incubator at 37°C and 5% carbon dioxide (CO₂).

Platelet-derived extracellular vesicle freeze-dried nebulized regardless of particle size group: The nebulized recovery liquid of the platelet-derived extracellular vesicle regardless of particle size dry powder solution and DMEM culture medium (containing 2% FBS) were mixed (volume ratio is 1:19) to prepare the platelet-derived extracellular vesicle freeze-dried nebulized culture medium regardless of particle size. Subsequently, the platelet-derived extracellular vesicle freeze-dried nebulized culture medium regardless of particle size was added to the wells of platelet-derived extracellular vesicle freeze-dried nebulized group regardless of particle size group and continually cultured for 18 hours in an incubator at 37°C and 5% carbon dioxide (CO₂).

Large-sized platelet-derived extracellular vesicle freeze-dried nebulized group: The nebulized recovery liquid of the large-sized platelet-derived extracellular vesicle dry powder solution and DMEM culture medium (containing 2% FBS) were mixed (volume ratio is 1:19) to prepare the large-sized platelet-derived extracellular vesicle freeze-dried nebulized culture medium. Subsequently, the large-sized platelet-derived extracellular vesicle freeze-dried nebulized culture medium was added to the wells of large-sized platelet-derived extracellular vesicle freeze-dried nebulized group, and continually cultured for 18 hours in an incubator at 37°C and 5% carbon dioxide (CO₂).

Medium-sized platelet-derived extracellular vesicle freeze-dried nebulized group: The nebulized recovery liquid of the medium-sized platelet-derived extracellular vesicle dry powder solution and DMEM culture medium (containing 2% FBS) were mixed (volume ratio is 1:19) to prepare the medium-sized platelet-derived extracellular vesicle freeze-dried nebulized culture medium. Subsequently, the medium-sized platelet-derived extracellular vesicle freeze-dried nebulized culture medium was added to the wells of medium-sized platelet-derived extracellular vesicle freeze-dried nebulized group, and continually cultured for 18 hours in an incubator at 37°C and 5% carbon dioxide (CO₂).

Small-sized platelet-derived extracellular vesicle freeze-dried nebulized group: The nebulized recovery liquid of the small-sized platelet-derived extracellular vesicle dry powder solution and DMEM culture medium (containing 2% FBS) were mixed (volume ratio is 1:19) to prepare the small-sized platelet-derived extracellular vesicle freeze-dried nebulized culture medium. Subsequently, the small-sized platelet-derived extracellular vesicle freeze-dried nebulized culture medium was added to the wells of small-sized platelet-derived extracellular vesicle freeze-dried nebulized group, and continually cultured for 18 hours in an incubator at 37°C and 5% carbon dioxide (CO₂).

The used medium of each group was removed. 90 µL of DMEM culture medium and 10 µL of CCK-8 buffer (DOJINDO, product number CK04) were added to each well, followed by continually culturing for 3 hours in an incubator at 37°C and 5% carbon dioxide (CO₂).

A multifunctional micro-plate spectrometer (model: Varioskan LUX^{™}, brand: Thermo Scientific^{™}) was used for analysis, and the absorbance value was set to 450 nm. A regression curve was established between absorbance value and cell number according to the CCK-8 buffer product instructions, and the absorbance value was then converted into the number of viable cells.

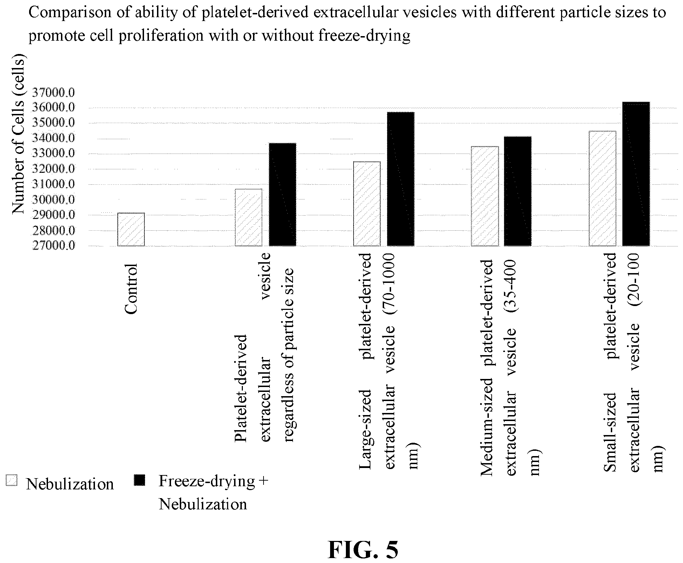

Experimental results are shown in Table 8 and FIG. 5.

**Table 8**

| | Control | Platelet-derived extracellular vesicle regardless of particle size | Large-sized platelet-derived extracellular vesicle (70-1000 nm) | Medium-sized platelet-derived extracellular vesicle (35-400 nm) | Small-sized platelet-derived extracellular vesicle (20-100 nm) |
|---|---|---|---|---|---|
| Nebulization | 29141.3 | 30717.5 | 32480.2 | 33480.0 | 34415.0 |
| Freeze drying+ Nebulization | | 33666.9 | 35728.8 | 34087.5 | 36347.5 |

### Example 5

### Comparison of ability of freeze-dried or non-freeze-dried platelet-derived extracellular vesicles to alleviate inflammation

The experimental steps are as follows. According to the preparation process in Example 1, four kinds of platelet-derived extracellular vesicle were prepared, and divided into two parts.

A portion of each of the four kinds of platelet-derived extracellular vesicle mentioned above was taken and freeze-dried in a volume of 1 mL to prepare four kinds of freeze and thaw activated platelet derived extracellular vesicle dry powder.

One portion of each of the four kinds of freeze and thaw activated platelet derived extracellular vesicle dry powder was taken and re-dissolved in 1 mL of normal saline to prepare four groups of freeze and thaw activated platelet derived extracellular vesicle dry powder solution.

Each group of platelet-derived extracellular vesicle was subjected to the nebulization procedure as described in Example 1 above using an aerosol generator (Pulmogine; brand HCmed), thereby obtaining multiple aerosols respectively. In this experiment, a centrifuge tube is used to collect the nebulized aerosols, the aerosols are gathered into a recovery liquid in the centrifuge tube, and subsequent experiments are carried out.

Experiments were performed using the same human nasal mucosal epithelial cells as in Example 2-1.

A 96-well plate is grouped into original wells, wells of LPS control, wells of platelet-derived extracellular vesicle nebulized group regardless of particle size, wells of large-sized platelet-derived extracellular vesicle nebulized group, wells of medium-sized platelet-derived extracellular vesicle nebulized group, and wells of small-sized platelet-derived extracellular vesicle nebulized group; wells of platelet-derived extracellular vesicle freeze-dried nebulized group regardless of particle size, wells of large-sized platelet-derived extracellular vesicle freeze-dried nebulized group, wells of medium-sized platelet-derived extracellular vesicle freeze-dried nebulized group, and wells of small-sized platelet-derived extracellular vesicle freeze-dried nebulized group, 3 replicates per well. Human nasal mucosal epithelial cells were added to the wells in each group, allowing each well contains the same number of cells and culturing in an incubator at 37°C and 5% carbon dioxide (CO₂) for 24 hours.

The used medium was removed. The cells in the wells of LPS control and experimental wells were suspended in DMEM culture medium containing 5 µg/mL LPS (containing 2% FBS), and the cells in the original wells were suspended in DMEM culture medium without LPS (containing 2% FBS), followed by culturing for 1 hour in an incubator at 37°C and 5% carbon dioxide (CO₂).

The used medium was removed and the following steps were performed.

Original wells: DMEM culture medium (containing 2% FBS) was added and continually cultured for 24 hours in an incubator at 37°C and 5% carbon dioxide (CO₂).

Platelet-derived extracellular vesicle nebulized regardless of particle size group: The platelet-derived extracellular vesicle nebulized recovery liquid regardless of particle size and DMEM culture medium (containing 2% FBS) were mixed (volume ratio is 1:19) to prepare the platelet-derived extracellular vesicle nebulized culture medium regardless of particle size. Subsequently, the platelet-derived extracellular vesicle nebulized culture medium regardless of particle size was added to the wells of platelet-derived extracellular vesicle nebulized group regardless of particle size group and continually cultured for 24 hours in an incubator at 37°C and 5% carbon dioxide (CO₂).

Large-sized platelet-derived extracellular vesicle nebulized group: The large-sized platelet-derived extracellular vesicle nebulized recovery liquid and DMEM culture medium (containing 2% FBS) were mixed (volume ratio is 1:19) to prepare the large-sized platelet-derived extracellular vesicle nebulized culture medium. Subsequently, the large-sized platelet-derived extracellular vesicle nebulized culture medium was added to the wells of large-sized platelet-derived extracellular vesicle nebulized group, and continually cultured for 24 hours in an incubator at 37°C and 5% carbon dioxide (CO₂).

Medium-sized platelet-derived extracellular vesicle nebulized group: The medium-sized platelet-derived extracellular vesicle nebulized recovery liquid and DMEM culture medium (containing 2% FBS) were mixed (volume ratio is 1:19) to prepare the medium-sized platelet-derived extracellular vesicle nebulized culture medium. Subsequently, the medium-sized platelet-derived extracellular vesicle nebulized culture medium was added to the wells of medium-sized platelet-derived extracellular vesicle nebulized group, and continually cultured for 24 hours in an incubator at 37°C and 5% carbon dioxide (CO₂).

Small-sized platelet-derived extracellular vesicle nebulized group: The small-sized platelet-derived extracellular vesicle nebulized recovery liquid and DMEM culture medium (containing 2% FBS) were mixed (volume ratio is 1:19) to prepare the small-sized platelet-derived extracellular vesicle nebulized culture medium. Subsequently, the small-sized platelet-derived extracellular vesicle nebulized culture medium was added to the wells of small-sized platelet-derived extracellular vesicle nebulized group, and continually cultured for 24 hours in an incubator at 37°C and 5% carbon dioxide (CO₂).

Platelet-derived extracellular vesicle freeze-dried nebulized regardless of particle size group: The nebulized recovery liquid of the platelet-derived extracellular vesicle regardless of particle size dry powder solution and DMEM culture medium (containing 2% FBS) were mixed (volume ratio is 1:19) to prepare the platelet-derived extracellular vesicle freeze-dried nebulized culture medium regardless of particle size. Subsequently, the platelet-derived extracellular vesicle freeze-dried nebulized culture medium regardless of particle size was added to the wells of platelet-derived extracellular vesicle freeze-dried nebulized group regardless of particle size group and continually cultured for 24 hours in an incubator at 37°C and 5% carbon dioxide (CO₂).

Large-sized platelet-derived extracellular vesicle freeze-dried nebulized group: The nebulized recovery liquid of the large-sized platelet-derived extracellular vesicle dry powder solution and DMEM culture medium (containing 2% FBS) were mixed (volume ratio is 1:19) to prepare the large-sized platelet-derived extracellular vesicle freeze-dried nebulized culture medium. Subsequently, the large-sized platelet-derived extracellular vesicle freeze-dried nebulized culture medium was added to the wells of large-sized platelet-derived extracellular vesicle freeze-dried nebulized group, and continually cultured for 24 hours in an incubator at 37°C and 5% carbon dioxide (CO₂).

Medium-sized platelet-derived extracellular vesicle freeze-dried nebulized group: The nebulized recovery liquid of the medium-sized platelet-derived extracellular vesicle dry powder solution and DMEM culture medium (containing 2% FBS) were mixed (volume ratio is 1:19) to prepare the medium-sized platelet-derived extracellular vesicle freeze-dried nebulized culture medium. Subsequently, the medium-sized platelet-derived extracellular vesicle freeze-dried nebulized culture medium was added to the wells of medium-sized platelet-derived extracellular vesicle freeze-dried nebulized group, and continually cultured for 24 hours in an incubator at 37°C and 5% carbon dioxide (CO₂).

Small-sized platelet-derived extracellular vesicle freeze-dried nebulized group: The nebulized recovery liquid of the small-sized platelet-derived extracellular vesicle dry powder solution and DMEM culture medium (containing 2% FBS) were mixed (volume ratio is 1:19) to prepare the small-sized platelet-derived extracellular vesicle freeze-dried nebulized culture medium. Subsequently, the small-sized platelet-derived extracellular vesicle freeze-dried nebulized culture medium was added to the wells of small-sized platelet-derived extracellular vesicle freeze-dried nebulized group, and continually cultured for 24 hours in an incubator at 37°C and 5% carbon dioxide (CO₂).

The cell culture medium in each well was transferred into a microcentrifuge tube. The interleukin (IL-6, an inflammatory factor) ELISA kit (human IL-6 ELISA kit) (Product No. ab178013, brand: Abcam) was used to detect the concentration of human IL-6 in the cell culture medium. A multifunctional micro-plate spectrometer (model: Varioskan LUX^{™}, brand: Thermo Scientific^{™}) was used for analysis, and the absorbance value was set to 450 nm. A regression curve was established between absorbance value and concentration of inflammatory factor IL-6 according to the interleukin (IL-6) ELISA kit product instructions, and the absorbance value was then converted into the concentration of inflammatory factor IL-6.

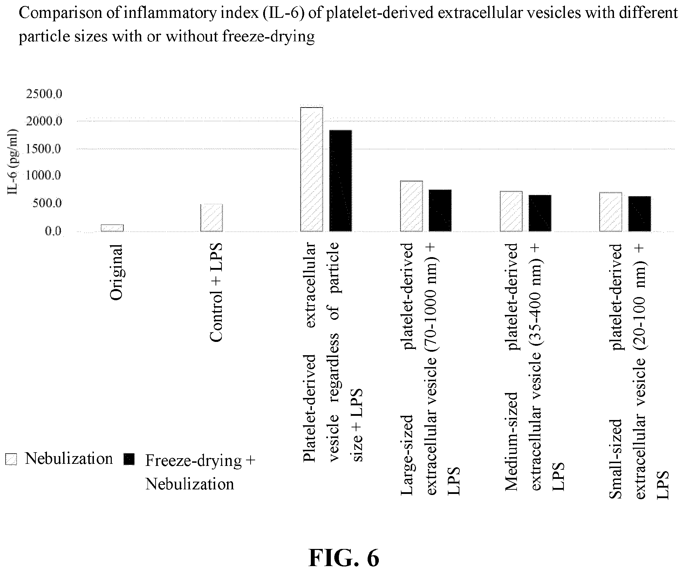

Experimental results are shown in Table 9 and FIG. 6.

**Table 9**

| | Original | LPS control | Platelet-derived extracellular vesicle regardless of particle size + LPS | Large-sized platelet-derived extracellular vesicle (70-1000 nm) + LPS | Medium-sized platelet-derived extracellular vesicle (35-400 nm) + LPS | Small-sized platelet-derived extracellular vesicle (20-100 nm) +LPS |
|---|---|---|---|---|---|---|
| Nebulizati on | 125.0 | 483.10 | 2255.70 | 921.00 | 712.20 | 702.70 |
| Freeze drying+ Nebulizati on | | | 1849.00 | 746.00 | 651.70 | 619.90 |

In summary, the present invention, as demonstrated by the results of the above examples, achieves the following technical effects: improving storage conditions; reducing side effects associated with chemically synthesized drugs, biologics, or cell-based therapies; addressing issues related to invasive treatments; overcoming usage limitations and regulatory restrictions of chemically synthesized drugs, biologics, or cell-based therapies; reducing the high costs of biologics or cell-based therapies; and improving the biological tolerance of natural biologics produced by cells and blood derivatives.

The means and improvements provided by the present invention are as follows. While chemically synthesized drugs, biologics, or cell-based therapies typically require strict storage conditions, the present invention can be stored under regular environmental conditions without special requirements. Chemically synthesized drugs and biologics are often artificially synthesized, raising concerns about potential side effects. In contrast, the present invention is a biologics product derived from cells and blood components, with no known side effects. Furthermore, while most chemically synthesized drugs, biologics, or cell-based therapies require invasive administration, the present invention is designed for inhalation, offering a non-invasive therapeutic approach. Conventional chemically synthesized drugs, biologics, and cell-based therapies can be difficult to use and any pose a risk of operational failure. In contrast, the present invention is easy to use and minimizes the risk of administration errors. Additionally, this biologic product can be efficiently mass-produced, thereby reducing costs. The present invention is a biologic product derived from cells and blood components, and has no problem of biological tolerance. Moreover, the present invention can be used in combination with other natural biologics produced from cells and blood derivatives to enhance therapeutic efficacy.

Although the present invention has been described with reference to the preferred embodiments, it will be apparent to those skilled in the art that a variety of modifications and changes in form and detail may be made without departing from the scope of the present invention defined by the appended claims.

## Claims

1. A pharmaceutical composition, comprising a freeze and thaw activated platelet-derived extracellular vesicle, wherein the freeze and thaw activated platelet-derived extracellular vesicle is processed by size exclusion chromatography (SEC) to have different particle sizes.

2. The pharmaceutical composition according to claim 1, wherein the freeze and thaw activated platelet-derived extracellular vesicle has a particle size ranging from 70-1,000 nm.

3. The pharmaceutical composition according to claim 1, wherein the freeze and thaw activated platelet-derived extracellular vesicle has a particle size ranging from 35-400 nm.

4. The pharmaceutical composition according to claim 1, wherein the freeze and thaw activated platelet-derived extracellular vesicle has a particle size ranging from 20-100 nm.

5. The pharmaceutical composition according to claim 1, wherein the freeze and thaw activated platelet-derived extracellular vesicle is further subjected to a lyophilization procedure, thereby transforming the pharmaceutical composition into a form of an inhaler.

6. The pharmaceutical composition according to claim 1, wherein the freeze and thaw activated platelet-derived extracellular vesicle is further subjected to a nebulization procedure, thereby transforming the pharmaceutical composition into a form of an inhaler.

7. The pharmaceutical composition according to claim 5, wherein the freeze and thaw activated platelet-derived extracellular vesicle is further subjected to a nebulization procedure, thereby transforming the pharmaceutical composition into a form of an inhaler.

8. A method for preparing the pharmaceutical composition according to claim 1, comprising the following steps:
(a) centrifuging a human whole blood sample to separate a plasma layer and remove leukocytes and erythrocytes, thereby obtaining platelet-rich plasma (PRP);
(b) centrifuging the platelet-rich plasma (PRP) to collect pellet and resuspending platelets in a solvent to obtain a platelet solution;
(c) subjecting the platelet solution to repeated freeze-thaw cycles to obtain a freeze and thaw activated platelet solution;
(d) centrifuging the freeze and thaw activated platelet solution and collecting supernatant to obtain a freeze and thaw activated platelet solution supernatant; and
(e) filtering the freeze and thaw activated platelet solution supernatant to obtain the freeze-thaw activated platelet-derived extracellular vesicle,
wherein the freeze and thaw activated platelet-derived extracellular vesicle is processed by size exclusion chromatography (SEC) to have different particle sizes.

9. The method according to claim 8, wherein the freeze and thaw activated platelet-derived extracellular vesicle has a particle size ranging from 70-1,000 nm.

10. The method according to claim 8, wherein the freeze and thaw activated platelet-derived extracellular vesicle has a particle size ranging from 35-400 nm.

11. The method according to claim 8, wherein the freeze and thaw activated platelet-derived extracellular vesicle has a particle size ranging from 20-100 nm.

12. The method according to claim 8, wherein the freeze and thaw activated platelet-derived extracellular vesicle is further subjected to a lyophilization procedure, thereby transforming the pharmaceutical composition into a form of an inhaler.

13. The method according to claim 8, wherein the freeze and thaw activated platelet-derived extracellular vesicle is further subjected to a nebulization procedure, thereby transforming the pharmaceutical composition into a form of an inhaler.

14. The method according to claim 12, wherein the freeze and thaw activated platelet-derived extracellular vesicle is further subjected to a nebulization procedure, thereby transforming the pharmaceutical composition into a form of an inhaler.

15. The pharmaceutical composition according to claim 1 for use in promoting wound healing of respiratory tract-related tissues and alleviating inflammation, wherein the pharmaceutical composition promotes wound healing of respiratory tract-related tissues by promoting cell migration and cell proliferation and alleviating inflammation.
